Europäisches Patentamt

**European Patent Office**

Office européen des brevets

(19)

(11) Publication number:

**0 280 576**
**A2**

# EUROPEAN PATENT APPLICATION

(12)

(21) Application number: 88301704.8

(22) Date of filing: 26.02.88

(51) Int. Cl.⁴: **C 07 K 7/06**
C 07 K 7/08, C 07 K 7/10,
C 07 K 1/00, A 61 K 39/40,
C 12 P 21/02, A 61 K 37/02,
C 12 P 21/00
// A61K39/09

(30) Priority: 27.02.87 GB 8704647

(43) Date of publication of application:
31.08.88 Bulletin 88/35

(84) Designated Contracting States: ES GR

(71) Applicant: COUNCIL OF GOVERNORS OF THE UNITED
MEDICAL AND DENTAL SCHOOLS OF GUY'S AND ST.
THOMAS'S HOSPITALS
London Bridge
London, SE1. (GB)

JOHNSON & JOHNSON CONSUMER PRODUCTS, INC.
501 George Street
New Brunswick New Jersey 08903 (US)

(72) Inventor: Lehner, Thomas
Department of Immunology Guy's Hospital
London SE1, 9RT (GB)

Haron, Jay A. Johnson & Johnson
Biotechnology Center Inc. P.O. Box 8289
La Jolla California 92038 (US)

Friedmann, Nadav Johnson & Johnson
Biotechnology Center Inc. P.O. Box 8289
La Jolla California 92038 (US)

(74) Representative: Goldin, Douglas Michael et al
J.A. KEMP & CO. 14, South Square Gray's Inn
London WC1R 5EU (GB)

(54) Synthetic anti-caries vaccine.

(57) Synthetic peptides are described, useful in the control of dental caries, and capable of provoking in vivo formations of antibodies that recognise Streptococcus mutans antigens.

An 11-mer is described together with larger peptides containing up to 35 amino acid units including the 11-mer and other sequences. These peptides and antibodies produced using the peptides as immunogens can be applied gingivally in primates to control dental caries.

EP 0 280 576 A2

Bundesdruckerei Berlin

0 280 576

## Description

The present invention concerns a peptide immunogen, partially or entirely of synthetic origin, of interest in the control of dental caries. In this Specification the term 'Streptococcus mutans' is to be understood as including Streptococcus sobrinus, since S. mutans serotypes d and g are now regarded by most workers in this field as a separate species designated S. sobrinus.

Dental caries can be reduced by immunising subjects with killed Streptococcus mutans, or purified immunogens from the cell wall of the bacteria, (T. Lehner, "Immunization Against Dental Caries", Vaccine, 3, 65-68 (1985)). The ultimate value of some of these immunogens is in doubt, because of antigens associated with S.mutans that are associated with tissue injury, (Stinson, M.W., Albini, B., and Nisengard, R.J., "Adverse Effects of Streptococcus mutans Antigens on Host Tissues", Molecular Microbiology and Immunobiology of Streptococcus mutans", 307-318, Edited by Hamada, S. et al., Elsevier Science Publishers B.V., Amsterdam, The Netherlands, (1986)). This invention represents an approach to production of an immunogen that is devoid of potential contamination by less desirable antigenic components of S.mutans.

A second advantage of this invention, and synthetic peptide vaccines in general, is that a relatively small molecule (or small number of molecules) can be synthesized that contain binding sites for protective antibodies as well as domains reacting with receptors on T-lymphocytes and associated molecules, (J. Brzofsky, "Antigenic Structural Requirements for MHC-Restricted Helper T-Cell Recognition", Modern Approaches to Vaccines, 21-23, Edited by R, Chanock, R. Lerner and F. Brown, Cold Spring Harbor Laboratory, Cold Spring Harbor, New York (1985)). This approach, when applied to a limited set of synthetic peptides, can provide resistance in a large population and minimize the problems associated with genetic restriction, (Milich, D.R. et al., "Immune Response to the Pre-S(1) Region of the Hepatitis B Surface Antigen (HbSAg): A Pre-S(1)-Specific T Cell Response Can Bypass Nonresponsiveness to the Pre-S(2) and S Regions of HbSAg", Journal of Immunology, 137: 315-322 (1986)).

Peptides of this invention may be regarded as derivatives of the protein Antigen I/II, (Russell, M.W., and Lehner, T., "Characterization of Antigens Extracted from Cells and Culture Fluids of Streptococcus mutans Serotype c", Archives of Oral Biology, 23: 7-15 (1978)). This protein is also referred to in the literature as B, IF, PI and SpaA (Russell, R.R.B., et al, "Characterization of Streptococcus mutans Surface Antigens" Molecular Mibrobiology and Immunobiology of Streptococcus mutans, 61-70, Edited by Hamada, S., et al, Elsevier Science Publishers B.V., Amersterdam, The Netherlands (1986)). Antigen I/II is a substance of molecular weight 175 Kd to 195Kd and is considered to contain two related antigenic materials, Antigen I of molecular weight 146-155 Kd and Antigen II of molecular weight 47 Kd to 49 Kd. These antigens are described in GB-A-2,060,247. Fragments of Antigen I or I/II of molecular weight 3.8 Kd, also known as Antigen X, are also known to be useful in the control of dental caries. (Giasuddin, A.S.M., Lehner, T., and Evans, R.W., "Identification, Purification and Characterisation of a Streptococcal Protein Antigen with a Molecular weight of 3800", Immunology, 50: 651-658 (1983) and European EP-A-0116472. Both Antigen I/II and Antigen X have been shown to be effective as vaccines, Antigen X being effective even when applied to the gingiva (Lehner, T., Mehlert, A., and Caldwell, J., Ibid.). In this Specification reference to molecular weight of peptides or proteins means molecular weight calculated by SDS-PAGE.

The present invention provides a synthetic peptide comprising a peptide having amino acid sequences identical or substantially identical to fragments of Antigen I or Antigen I/II or Antigen X, the peptide having amino acid sequences identical or substantially identical to fragments of Antigen I or Antigen I/II or Antigen X being capable of provoking formation in vivo of antibodies that recognise S. mutans antigen (SA). In one embodiment the peptide having amino acid sequences identical or substantially identical to fragments of Antigen I or Antigen I/II or Antigen X has a molecular weight less than 3.8 Kd.

The peptides of the invention can be produced by organic synthesis, recombinant DNA technology, or purified from S. mutans, e.g. by degradation of antigen I/II or Antigen X. The peptides can be either linked to a carrier, or not. The immunogenicity of the peptides can be enhanced by combining with an adjuvant or other immunomodulators. The peptide sequence must contain amino acid sequences analogous to those found in Antigen I/II or Antigen X but can also contain sequences that modify the peptide structure or immunogenicity.

The present invention provides peptides for vaccine production, the peptide being preferably a synthetic peptide that directs production of specific anti-Streptococcus mutans antibodies and stimulates helper T-lymphocyte proliferation to combat dental caries.

The peptide of the present invention does not contain the whole of naturally occurring Antigen I/II from Streptococcus mutans. Instead it consists of one, or more, polypeptides regions corresponding to a limited region or number of regions of Antigen I/II optionally with other sequences that function to enhance immunogenicity. A vaccine based on the peptide of this invention is normally free of naturally occurring S. mutans molecules, but this is not essential. The peptide of the invention can also contain regions of, molecules derived from, or synthetic analogues of other pathogens such as tetanus, Hepatitis B Virus, or others.

The peptide of the invention can be of different molecular weights, depending on how it is produced. For example, the peptide produced by chemical synthesis is preferably limited to 50 or fewer amino acids. The same peptide can be produced as part of a larger protein when produced by recombinant DNA methods. In this conjugate protein embodiment, the peptide is usually smaller than Antigen I/II or Antigen X, but can be

2

larger than the latter.

In one aspect, the peptide of the present invention comprises a synthetic peptide sequence, preferably of molecular weight less than 3.8 Kd, for example containing about 6 to about 50 amino acid units and including the antigenic determinants present in Antigen X so that the peptide sequence of the present invention is capable of provoking in vivo formation of antibodies that will recognise, neutralise and protect against S. mutans. It is particularly preferred that the peptide sequence of the present invention should comprise no more than about 25 amino acid units and our investigations have now reached the stage where we have been able to identify amino acid sequences comprising between about 10 to about 24 amino acids which include the necessary antigenic determinants. Sequencing work on Antigen X indicate that it probably has the following sequence I:

| Gly: | Ala: | Val: | Asp: | Ser: | Ile: | Leu: | Gly: |
|------|------|------|------|------|------|------|------|
| 1 | 2 | 3 | 4 | 5 | 6 | 7 | 8 |

| Gly: | Val: | Ala: | Thr: | Tyr: | Gly: | Ala: | Ala: |
|------|------|------|------|------|------|------|------|
| 9 | 10 | 11 | 12 | 13 | 14 | 15 | 16 |

Position 1 may be Ala. Position 2 may be Gly. Position 14 may be Ala or Ile. Position 15 may be Ser. Position 16 may be Glu.

The synthetic peptide of the invention will be or include sequence I or will be or include an immunologically effective fraction of sequence I or will be or include an immunologically equivalent sequence to sequence I or fraction thereof. For example, the immunological properties of sequence I can be retained even when Asp is replaced by Glu, when Leu is replaced by Val or Ile, when Tyr is replaced by Phe or Trp, when Ile or Ala or Leu is replaced by Val or Leu, when Val is replaced by Ile or when Thr is replaced by Ser.

As explained below, sequence I or fragments thereof or immunological equivalents of sequence I or fragments thereof can be built into larger molecules, in accordance with the invention, by linking at either or both the carboxy and amino terminus via cysteine links bonded to the end where growth is required. This technique enables the peptide of the invention to be linked to additional amino acid sequences so that, for example, it assumes in solution a conformation that more closely mimics Antigen I/II.

Alternative bonding cysteine to both ends enables the molecule to be cyclised.

Another type of amino acid sequene to which the peptide of the invention can be linked by the techniques discussed are sequences including another antigenic determinant from S. Mutans or from other pathogens so that a polyvalent vaccine can be produced.

As indicated above, it is frequently desirable to conjugate the synthetic peptide sequence of the invention with a further peptide sequence so as to render the peptide sequence of the invention immunogenic. The conjugation may be carried out by methods known per se and, where conjugation is to be carried out by chemical methods, the peptide sequence of the invention including the antigenic determinants can be bonded by conventional peptide-forming methods to large peptide sequence such as bovine serum albumin, keyhole limpet hemocyanin, tetanus or diphtheria toxoids and M.tuberculosis purified protein derivative (PPD) or to a synthetic adjuvant such as muramyl dipeptide.

When the synthetic peptide sequence of the invention is bonded to a carrier molecule in the manner indicated above, then the resulting molecule may be regarded as "semi-synthetic" in that the antigenic determinant containing part of the molecule is synthetic while this synthetic fragment is bonded synthetically to a larger fragment of natural origin.

Alternatively, if the whole conjugate is to be prepared by genetic engineering techniques, it is possible to clone a DNA encoding the desired "synthetic" fragment of up to perhaps 50 amino acid units including the desired antigenic determinants and further carrier peptide so that, when the cloned DNA fragment is expressed from a transformed host cell, the expressed peptide can be identical to the peptides prepared as described above by chemical conjugation of small synthetic fragments containing the antigenic determinant with larger fragments of natural origin but, as in the case described above, regardless of whether the conjugate is prepared by genetic engineering techniques or by chemical conjugation, it is to be regarded as "synthetic" in accordance with the present invention.

As an alternative to bonding the relatively small amino acid sequences of the invention including the antigenic determinants, it is possible to produce molecules of large molecular weight in accordance with the present invention by the polymerisation of monomeric peptide sequences including the antigenic determinants. Such polymerisation may link two or more, e.g. 2 to 100 peptide sequences including the antigenic determinant and may be accomplished by methods known per se and used for instance in cyclising peptides or in cross-linking peptides to carriers. Conveniently the polymerisation is conducted in a suitable buffer either using glutaraldehyde or by formation of sulphydryl bridges between cysteine residues (which may be added to the termini of the monomers if necessary).

The peptide sequences of the invention, incorporating the antigenic determinant, may be used in vaccine preparations for the control of dental caries. Such vaccine compositions may include the peptide of the invention in effective amounts together with appropriate inert carriers. The peptide of the invention can be used in any of the forms described above, e.g. as monomeric molecules, polymeric molecules conjugated with polypeptide carrier molecules or immunomodulators. Permeabilizers and other compounds (e.g., protease inhibitors) can also be formulated as part of the invention.

It is often necessary to include additional compounds in the formulation of a synthetic peptide vaccine. For example, the invention, in one of its forms, is linked to a carrier protein, e.g. tetanus toxoid, which serves to enhance its immunogenicity. Carriers such as tetanus toxoid are effective and approved for human use. In addition, the immunogen is formulated to contain an adjuvant that also enhances the immune response. Often the adjuvant is simply aluminium hydroxide and the protein is merely adsorbed to it. One alternative is the synthetic adjuvant, muramyl dipeptide. Adjuvants are plentiful, and this invention is not limited by the nature of the carrier or adjuvant.

The peptides of the invention or vaccinating conpositions containing them can be used in the control of dental caries by administering the peptide of the invention or a composition containing it to a host. Various methods of administration may be used including parenteral administration or oral or topical administration. Where the material is to be administered parenterally, it may be administered by for example, intramuscular injection in which case a dose of 0.1 to 1mg for instance about 0.5mg in saline and using aluminium hydroxide adjuvant, repeated several times, may be appropriate. The preferred method of administration is by topical administration, particularly through the gingival epithelium or the oral mucosa or by swallowing the peptide. For this purpose, the peptide of the invention may be formulated in simple solutions or as emulsions, dispersions, pastes, gels or aerosols. Ideally, for ease of administration, the polypeptides of the invention can be formulated in toothpaste chewing gum or lozenge or other material for cleaning or coating teeth or in mouthwashes or other materials for cleaning the mouth.

The peptides of the invention may also be used for the production of antibodies. Such antibodies can be used either for diagnostic purposes or for passive immunisation of a host to provide a measure of protection against S. mutans infection. They may also be used in purification of the peptide for instance by affinity chromatography.

The peptides of the invention can be used for the production of polyclonal or monoclonal antibodies. Polyclonal antibodies may be prepared by any of the conventional methods for antibody production essentially involving the immunisation of an appropriate host animal which may be any one of the large or small laboratory animals conventionally used for antibody production. It is normally necessary for the peptides of the invention to be used in immunogenic form, for example in the form in which they are conjugated with larger carrier polypeptie molecules and, after immunisation of the host animal, blood or other body fluids can be removed from the host animal and the antibodies recovered from the blood or plasma by methods known per se.

Where monoclonal antibodies are required, then an immunogenic form of the peptide of the invention can be used for the in vivo immunisation of an appropriate host or the in vitro stimulation of B-lymphocytes, spleen cells taken from the host or stimulated B-lymphocytes are then hybridised with myeloma cells by methods known per se to provide a population of hybridomas from which appropriate hybridomas can be selected and maintained for the production of monoclonal antibodies. One appropriate host species is the mouse.

Monoclonal antibodies against the synthetic peptide of the invention, especially monoclonal antibodies against the 21 mer of Example 1 below constitute particular aspects of the present invention.

## DEFINITIONS AND ABBREVIATIONS USED IN THIS SPECIFICATION

Amino Acid One and Three Letter Codes
A Ala Alanine      L Leu Leucine
R Arg Arginine     K Lys Lysine
N Asn Asparagine     M Met Methionine
D Asp Aspartic Acid     F Phe Phenylalanine
C Cys Cysteine     P Pro Proline
Q Gln Glutamine     S Ser Serine
E Glu Glutamic Acid     T Thr Threonine
G Gly Glycine     W Trp Tryptophan
H His Histidine     Y Tyr Tyrosine
I Ile Isoleucine     V Val Valine

Synthetic peptide - (a) Sequence of amino acids synthesized by chemical methods, i.e. building up from smaller amino acid sequences or single amino acids, see for example Merrifield, et al, J. Am Chem. Soc., 85, 2149 -(1963), Kent, S.B.H., Biomedical Polymers eds., E.P. Goldberg and A. Nakajima, (Academic Press, New York) 213-242 (1980), or (b) - a sequence (a) bonded by chemical means to another amino sequence which is also a type (a) sequence or is a naturally occuring or biosynthesised sequence or a fragment thereof or (c) a sequence produced by genetic engineering techniques from a transformed host cell.

Immunomodulator - Any molecule that alters the immune response to a peptide by direct or indirect effect on T-or B-lymphocytes.

## Description of the Drawings

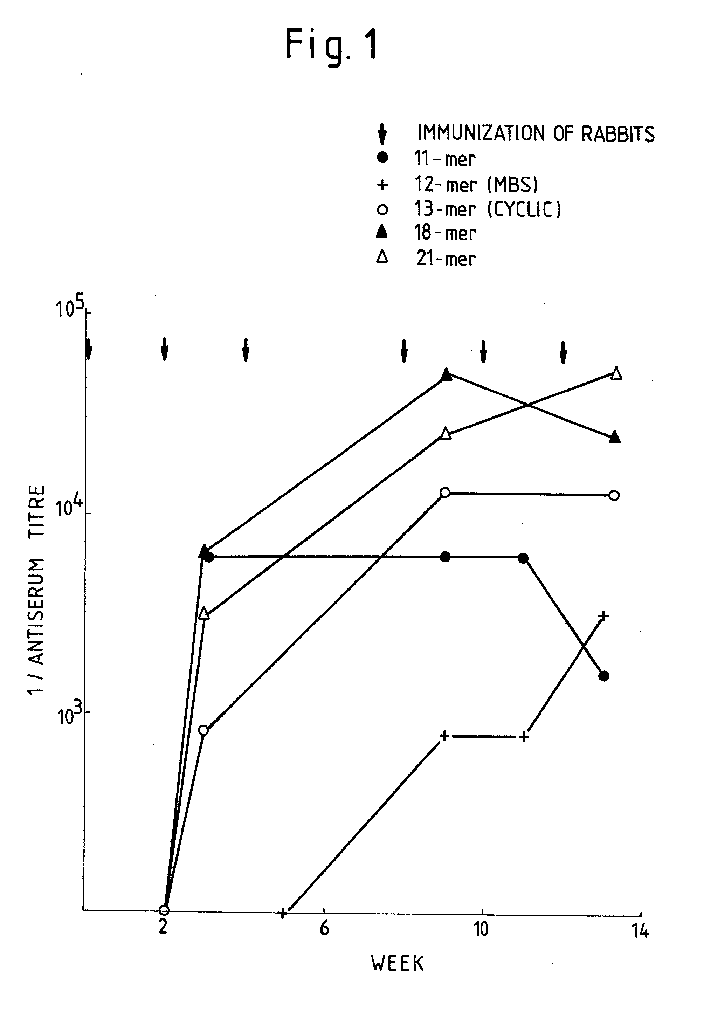

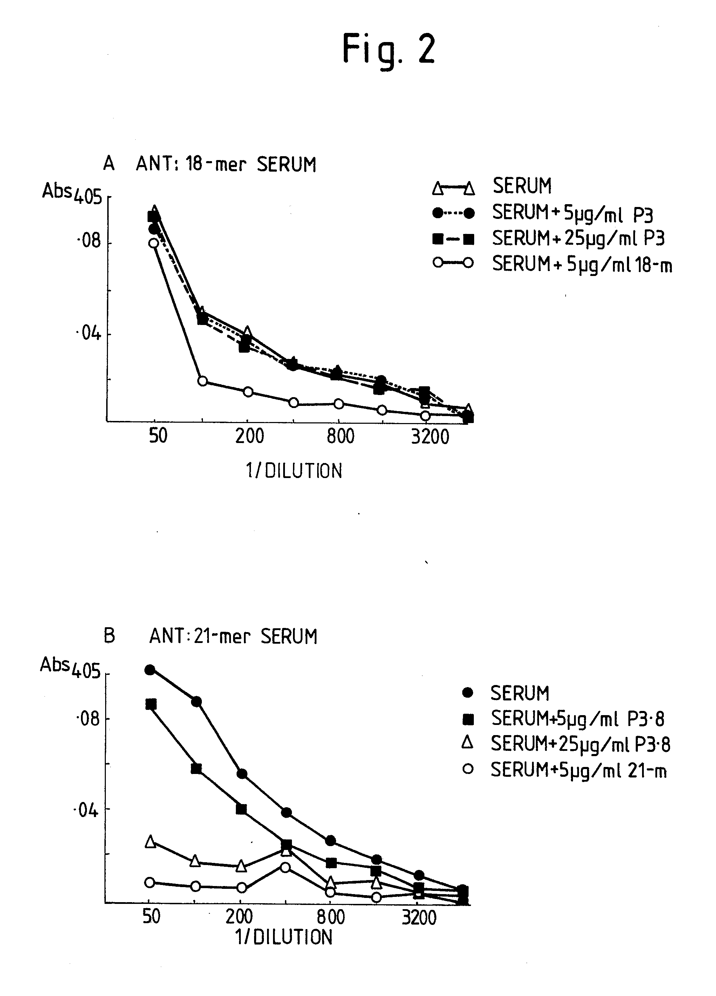

Figures 1 and 2 show results of assays, described in detail in Examples below, to demonstrate the immunogenicity of synthetic polypeptides of the invention.

Fig. 1 shows the results of serum assays on rabbits immunised with peptides according to the invention. The ordinate is the reciprocal of the log of the highest dilution of an antiserum that reacts with peptide (see Example 5). The abscissa is the number of weeks after primary immunisation. Arrows denote injections with adjuvented peptide linked to carrier.

Fig. 2 shows the results of assays to demonstrate the specificity of antisera against 18-mer (above) and 21-mer (below). The ordinate is the extent of immunoglobulin binding, as determined by a colour change when ABTS is added to the ELISA well (see Example 5). The absicssa is the reciprocal of the dilution of the antiserum-peptide mixture.

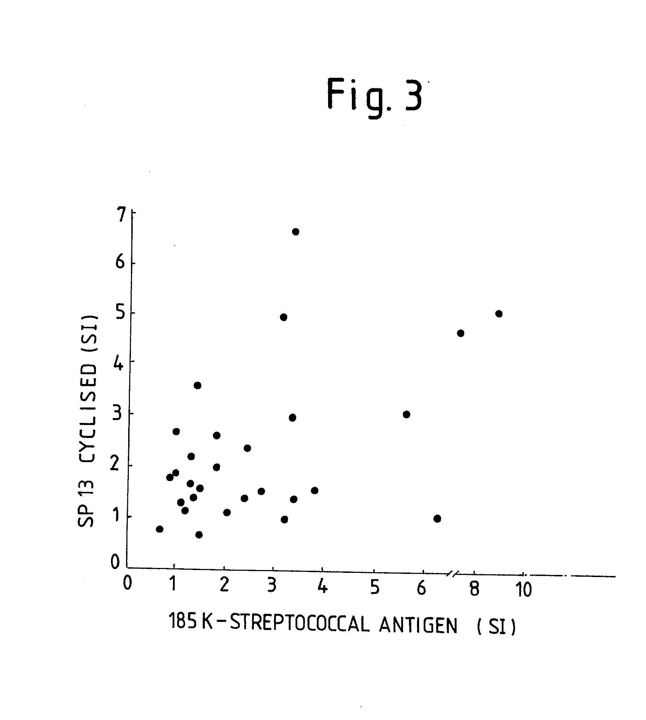

Fig. 3 is a graph showing the statistically significant ($r = 0.5018$, df 26, $p < 0.01$) correlation between the lympho-proliferative responses of human T4 cells when stimulated with 185K streptococcal antigen (abscissa) or synthetic peptide (13-mer, cyclised), (ordinate). The results are expressed as stimulation indices (SI).

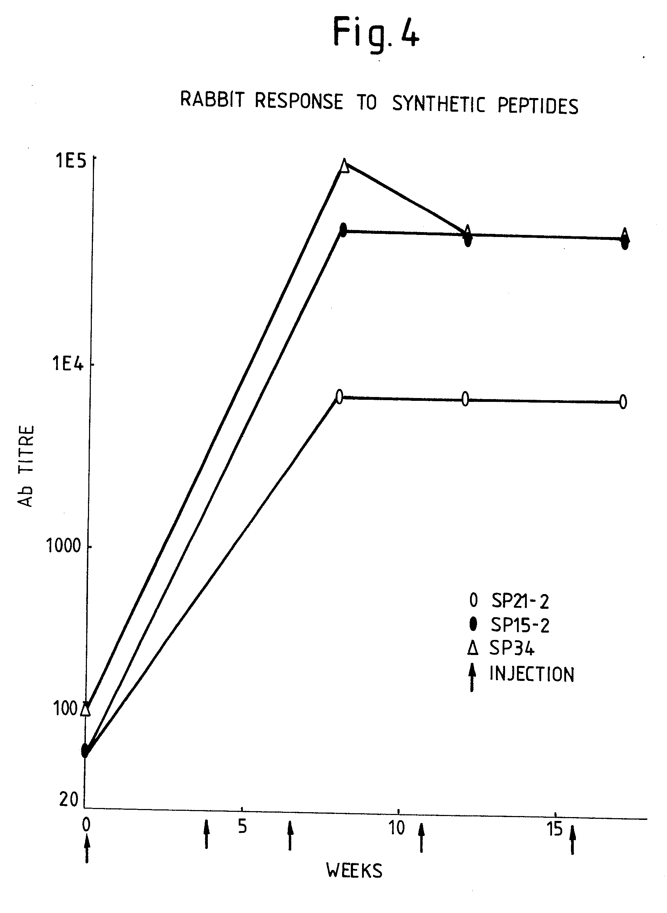

Figs. 4-6 show results obtained in experiments described in Examples 13 and 14 below.

The present invention is illustrated by the following Examples.

## EXAMPLE 1

Synthesis and Characterisation of a 21-mer (SP21) GAGAGAVDSILGGVATYGAAC

Boc-(4MeBz)-cysteine esterified to phenylacetamidomethyl (PAM) resin was purchased from Applied Biosystems Inc. Synthesis of the 21 amino acid peptide was accomplished using the symmetrical anhydride adaptation of the classical Merrifield solid-phase peptide synthesis chemistry (Hagenmaier, H., and Frank, H. [1972], Hoppe-Seyler's Z. Physiol. Chem. 353, 1973-6, Merrifield, R.B., [1963], J. Am. Chem. Soc. 85, 2149-54). The peptide sequence GAGAGAVDSILGGVATYGAAC was synthesised on an Applied Biosystems Inc. Model 430A automated peptide synthesiser using the symmetrical anhydride coupling programme. 0.5 Mmole of Boc-(4MeBz)-cysteine was coupled with symmetric anhydride amino acid at a two-fold excess over the reactive amino group.

### Amino Acid Derivatives

Boc-Gly     Boc-Ala
Boc-Val     Boc-(0-Bzl)-Asp
Boc-(0-Bzl)-Ser     Boc-Ile
Boc-Leu     Boc-(0-Bzl)-Thr
Boc-(0-BrZ)-Tyr     Boc-(4-MeBz)-Cys

The peptide was cleaved from the resin with hydrofluoric acid containing 10% anisole and extracted with 50% acetic acid. The hydrofluoric acid treatment also served to remove the protecting groups. These groups are:

Boc: tert-butyloxycarbonyl protecting group for the alpha amino group on the amino acid. It is removed by hydrolysis in 50% trifluoroacetic acid (TFA); 50% dichloromethane after the amino acid has been coupled to the growing peptide. This frees the primary amine to react in the subsequent condensation.

0-Bzl: Benzyl ester, attached to serine, threonine, and aspartic acid to protect the hydroxyl or carboxyl side-chain during synthesis.

9-BrZ: ortho bromobenzyloxycarbonyl, attached to the phenolic hydroxyl group of tyrosine to protect it from alkylation during synthesis.

4-MeBz: 4-Methylbenzyl: bound to the sulfhydryl group of cysteine to prevent oxidation.

Anisole (methyl phenyl ether) serves as a scavenger for nucleophiles during HF cleavage and after the protecting groups have come off of the peptide.

The solution was filtered to remove resin and chromatographed on a Fractogel TSK HW-40F gel filtration column using a mobile phase of 50% aqueous acetic acid. 10 ml. fractions were collected and those that absorb 280nm ultraviolet light were lyophilised. The peptide was characterised by amino acid analysis on a Beckman 6300 amino acid analyser and shown to be the 21-mer of the formula indicated above. Analytical high pressure liquid chromatography was performed on a 250 x 4.5 mm Vydac C-4 reverse-phase column using a gradient of 10-50% acetonitrile in 0.1% aqueous TFA over 20 minutes. The flow rate was 1.0 ml/min. Peptide elution was monitored at a wavelength of 214 nm and all preprarations of the material showed a major peak that contained >60% of the UV-absorbing material.

## EXAMPLE 2

CVDSILGGVATYC

### Synthesis and Characterisation of Cyclic 13-mer

Synthesis of this peptide was by a similar procedure to that in Example 1. The starting material was Boc-(4MeBz)-Cys esterified to PAM resin and the subsequent amino acids were added to the peptide chain using the same symmetric anhydride chemistry. The protecting groups on all of the amino acids used were as described in Example 1. The order of addition was YTAVGGLISDVC.

After chromatography and lyophilisation, the peptide was cyclised as follows: 0.1 mg of peptide was dissolved in 0.1M ammonium bicarbonate (pH = 7.8) and allowed to stir overnight at room temperature. Material before and after incubation were tested for the presence of free cysteine residues by the spectroscopic method of Ellman (Ellman, G.L. [1959] Arch. Biochem. Biophys. 82:70). The peptide was then lyophilised and chromatographed on a Fractogel TSK HW-40F to remove salt. The peptide was lyophilised and an aliquot was resuspended in 0.4 ml of deionised distilled water. The solution was made 1.25% 2-mercaptoethanol (v/v) and brought to pH = 7.8 with 1M ammonium bicarbonate. This reduced material, and the starting material were then analysed by HPLC to confirm that the peptide was cyclised and subsequently linearised.

## EXAMPLE 3

VDSILGGVATY

### Synthesis and Characterisation of Linear 11-mer

The general procedure of Example 1 was followed but using the following reagents. Boc (o-Bromocarbobenzoxy)-tryosine-PAM was purchased from Applied Biosystems and the amino acids TAVGGLISDV were added stepwise by the same procedure as described in Example 1. The o-bromocarbobenzoxyl (BromoCBz) group was removed by hydrofluoric acid in a way analogous to that for Boc-(o-Brz) group removal in Examples 1 and 2. For some studies, the lyophilised peptide was resuspended in 50% acetic acid, 0.1% TFA and further purified by reverse-phase HPLC. Chromatography conditions were the same as for the analytical runs described in Examples 1 and 2, only multiple runs were made and the major peak pooled. The pooled material was rechromatographed and the major peak, which eluted at 9 minutes (28% acetonitrile) contained 93% of the UV-absorbing material.

## EXAMPLE 4

### Linkage of the Peptide to Carriers

The peptide prepared in Examples 1, 2 and 3 were linked to large proteins for two reasons, so that the protein could serve as an immunologic carrier, allowing vaccinated hosts to generate an appropriate immune response against peptide and to cause the peptide to adsorb to microtitre plates quantitatively for immunologic assays.

### Procedure 1 for cross-linking

This is based on an adaptation of the procedure of Avrameas (Immunochemistry [1969] 6, 43-52). 5.0 mg of peptide and 5.0 mg of carrier protein were dissolved in 0.7 ml of 0.25 M potassium phosphate buffer, pH = 8.0. 4 ul of 25% glutaraldehyde was added and the solution was incubated at room temperature for 30 minutes. Free peptide and salt were removed by extensive dialysis in membranes with a 4,000 or 6,000 molecular weight cut off.

The dialysis buffer was 0.05 M sodium phosphate buffer, pH = 6.0 and an aliquot removed for amino acid analysis.

### Procedure 2 for cross-linking

This is based on an adaptation of the procedure of Liu, F-T et al. (Biochemistry [1979], 18, 690-7). 4 mg. of carrier protein dissolved in 0.25 ml 0.01 M sodium phosphate buffer was mixed with 0.085 ml of 6 mg/ml m-Maleimidobenzoyl-N-hydroxysuccinimide ester (MBS) in dimethylformamide. Incubation, with stirring, was carried out for 30 minutes at room temperature. It was then chromatographed through a 2.5 x 25 cm column of Biogen P30 using 0.05 M sodium phosphate buffer, pH = 6.0 as the mobile phase and the material eluted in the void volume collected.

This material was brought to a final volume of 8.0 mls and 1 ml of the same buffer containing 5 mg of peptide was added. The peptide can first be tested for free cysteine groups by the Ellman procedure cited above. The pH of the solution was adjusted to neutral with sodium hydroxide and the cross-linkage allowed to take place over 3 hours at room temperature. Particulates and aggregates were removed by centrifugation, and protein concentration determined by standard laboratory procedures. The extent of the cross-linking can be determined by Ellman assay before and after linkage.

## EXAMPLE 5

### Procedure for measuring antipeptide antibodies

### Procedure 1- ELISA Assay

The assay system described below is adapted from the guidelines of Kurstak (Bulletin of the World Health Organisation [1985], 53, 793-811). Assay plates were produced by filling each well of a 96-well microtitre plate with 500 ng of peptide linked to ovalbumin (by procedure 1) in 0.1 ml of phosphate buffered saline. The protein was allowed to adsorb by drying the plate overnight at 37°C. The plates were washed with phosphate buffered saline and stored at -20°C until use. Non-specific sticking of immunoglobulin was prevented by treating the wells with 0.1 ml of mT-wash fr 1 hour at 37°C. The mT-wash was removed and the plates blotted dry. The wells were filled with 0.1 ml mT-wash and antiserum was serially diluted in the wells containing mT-wash. Antibodies were allowed to bind to the adsorbed antigen for one hour at 37°C. At that time the wells were aspirated and washed 5 times with PBS containing 0.5% Tween 20. The plates were blotted dry and refilled with 0.1 ml of mT-wash containing a 1:4000 dilution of goat anti-rabbit Ig (G+M+A) that has been conjugated with horse radish peroxidase. The second antibody was allowed to react with antigen bound immunoglobulin for 1 hour at 37°C. The plates were aspirated and the wells washed 5 times with PBS + Tween 20 and blotted dry. Extent of peroxidase linked immunoglobulin binding was quantified with 2,2'-azino-di-[3-ethyl-benzthiazoline sulphonate] (ABTS) under conditions recommended by the manufacturer (Kirkegaard & Perry Laboratories, Inc., Gaithersburg, Maryland, USA) and read at 495 nm on a BioTek EL-310 ELISA plate reader (BioTek Instruments, Burlington, Vermont, USA). Titres were the reciprocal of the greatest dilution that clearly gave a positive reading in the titration.

Phosphate Buffered Saline (PBS)
0.15 M Sodium chloride
0.01 M Sodium phosphate
pH = 7.2
mT-wash
5% Newborn calf serum
1% Bovine serum albumin
0.5% Tween 20
in Phosphate buffered saline

## EXAMPLE 6

### Immunisation with synthetic peptide

The synthetic peptides listed in Examples 1 to 3 were conjugated to tetanus toxoid using glutaraldehyde as the cross-linking agent. The material was dissolved in PBS and 100 micrograms was brought to 0.5 ml and mixed with an equal volume of Complete Freund's Adjuvant (CFA). The mixture was emulsified and immediately injected, subcutaneously, into 4 sites on the back of a New Zealand White rabbit. Subsequent injections were administered in a similar fashion, only with half the amount of protein and in Incomplete Freund's Adjuvant (IFA).

Two rabbits were immunised with each peptide. In all cases except for the 12-mer (VDSILGGVATYC) the carrier was tetanus toxoid. For the 12-mer the carrier was MBS linked to keyhole limpet hemocyanine. Periodically, serum was taken from each rabbit and assayed, by ELISA, to determine the production of antipeptide antibodies.

The assay plate wells contained the same peptide as was used to immunize the rabbit. Each peptide was glutaraldehyde linked to ovalbumin prior to attachment to the ELISA plate in order to ensure all peptides bound equally well to the plastic. Results are shown in Fig 1.

The poorest immunogens were the 11-mer and 12-mer. Although the 12-mer took longer to induce a response, the final response appeared stronger than that from the 11-mer. Cyclic 13-mer showed a superior response, with titres of >10,000 after the fourth injection. The two largest peptides, the 18-mer (GAVDSILGGVATYAAALC) and 21-mer showed the higher persistent titres than any of the smaller peptides.

## EXAMPLE 7

### Determination of the specificity of Antipeptide Antisera

Specificity of the antipeptide immune response was demonstrated by measuring the ability of the antipeptide antibodies to bind Antigen X adsorbed to an ELISA well. Specificity was confirmed by allowing the antipeptide antisera first to bind antigen (either Antigen X or the synthetic peptide) and then incubating it in an ELISA plate containing Antigen X. If the synthetic peptide is a valid mimic for AntigenX, then this binding will be blocked by pretreatment of the antisera with either Antigen X or synthetic peptide.

Pretreatments were performed in a final volume of 0.2ml of mT-wash and contained 5 or 25 micrograms of Antigen X. Pretreatment was for sixteen hours at four degrees. The mixtures were then diluted serially onto ELISA plates containing 500 ng of Antigen X per well. The assay was carried out in the same manner as described in Example 5. The results are shown in Fig. 2.

The top graph in Fig. 2 shows that although the antipeptide antiserum binds Antigen X on the plate, preincubation with Antigen X does not inhibit this process. In the case of the synthetic 18-mer binding is greatly reduced. This shows that the antibodies recognise synthetic peptide far better than they recognise Antigen X. The bottom panel shows the results when anti-21-mer is used. In this case, binding to the Antigen X on the plate is inhibited by preincubation with either Antigen X or synthetic peptide. This suggests that immunisation with 21-mer causes production of antibodies that recognize Antigen X in a highly specific manner.

EXAMPLE 8

Human T4 cells were stimulated with 185K strepotococcal antigen or the 13-mer of Example 2 and the lymphoproliferative responses measured. Results are shown graphically in Fig. 3. The results in Fig. 3 show a significant positive correlation in stimulation of t-cells by the 185K-SA and the 13-mer (P less than 0.01).

EXAMPLE 9

Rhesus monkeys were immunised against Antigen X or the 13-mer of Example 2 administered in DMSO. Antibody titres in saliva, gingival, crevicular fluid and serum were measured. The results are given in Table 1 and show that local immunisation with the cyclic 13-mer induces an immune response as demonstrated by the presence of specific antibodies in both saliva and gingival fluid.

Antibodies in saliva, gingival fluid and serum after gingival immunization with cyclised SP 11 in DMSO

| Antibodies | Anti-SP 13 cycl. Antibodies (Net cpm) Weeks | | | | | | | | | | Anti- X -SA Antibodies (Net cpm) Weeks | | | | | | | | | |
|---|---|---|---|---|---|---|---|---|---|---|---|---|---|---|---|---|---|---|---|---|
| | 0 | 2 | 3 | 4 | 5 | 7 | 12 | 17 | 24 | 25 | 0 | 2 | 3 | 4 | 5 | 7 | 12 | 17 | 24 | 25 |
| Salivary IgA | 0 | 8 | 17 | 26 | 20 | 16 | 6 | 80 | 35 | 45 | 0 | 1 | 13 | 50 | 0 | 5 | 1 | 47 | 32 | 22 |
| Gingival IgG | 0 | 6 | 0 | 0 | 24 | 50 | 6 | 14 | 12 | 30 | 0 | 0 | 0 | 0 | 0 | 9 | 0 | 16 | 32 | 36 |
| Serum IgG | 0 | 5 | 9 | 0 | 0 | - | 115 | 0 | 130 | 0 | 0 | 0 | - | 50 | - | - | 70 | 0 | 82 | 0 |

TABLE 1

EXAMPLE 10

Synthesis and Characterization of Dimer 15-mer (SP15-2)

```
VDSILGGVATYGAAC┐
VDSILGGVATYGAAC┘
```

Synthesis of this peptide was by a similar procedure to that in Example 1. The starting material was Boc-(4MeBz)-Cys esterified to PAM resin and the subsequent amino acids were added to the peptide chain using the same symmetric anhydride chemistry. The protecting groups on all of the amino acids used were described in Example 1, and removed in the same way. The order of addition of the amino acids was AAGYTAVGGLISDV.

After chromatography and lyophilisation, the peptides were dimerised as follows. Peptide was dissolved in 1.0 ml of 0.1M ammonium bicarbonate (pH = 7.8) for every 1.0 mg of peptide monomer and allowed to stir overnight at room temperature. Material before and after incubation were tested for free cysteine by the Ellman method described in Example 2. The peptide was then lyophilised and chromatographed on a Fractogel TSK HW-40F column to remove salt. The peptide was lyophilised and an aliquot was resuspended in 0.4 ml of deionised distilled water. The solution was made 1.25% 2-mercaptoethanol (v/v) and brought to pH = 7.8 with 1M ammonium bicarbonate. This reduced material, and the starting material were then analysed by reverse-phase HPLC, under the same conditions as in Example 1, to confirm that the peptide was dimerised and subsequently linearised.

EXAMPLE 11

Synthesis and Characterisation of Dimer 17-mer (SP-17-2) and Dimer 21-mer (SP21-2)

```
GAVDSILGGVATYGAAC          GAGAGAVDSILGGVATYGAAC
             |                          |
GAVDSILGGVATYGAAC          GAGAGAVDSILGGVATYGAAC


   SP17-2                         SP21-2
```

Synthesis of these peptides was by a similar procedure to that in Example 1. The starting material was Boc-(4MeBz)-Cys esterified to PAM resin and the subsequent amino acids were added to the peptide chain using the same symmetric anhydride chemistry. The protecting groups on all of the amino acids used were described in Example 1, and removed in the same way. The order of addition of the amino acids was AAGYTAVGGLISDVAG for SP-17-2 and AAGYTAVGGLISDVAGAGAG for SP21-2.

After chromatography and lyophilisation, the peptides were dimerised in the same way as Example 10. This peptide was then tested by free cysteine and by reduction followed by HPLC as described above.

EXAMPLE 12

Synthesis and Characterisation of 34-mer (SP-34)

GAVDSILGGVATYGAAGAVDSILGGVATYGAALC

Synthesis of this peptide was by a similar procedure to that in Example 1. The starting material was Boc-(4MeBz)-Cys esterified to PAM resin and the subsequent amino acids were added to the peptide chain using the same symmetric anhydride chemistry. The protecting groups on all of the amino acids used were described in Example 1, and removed in the same way. The order of addition of the amino acids was LAAGYTAVGGLISDVAGAAGYTAVGGLISDVAG. Purification and characterisation were the same as for linear 21-mer (Example 1).

EXAMPLE 13

Rabbits were immunised against the dimer 15-mer (SP15-2) the dimer 21-mer (SP21-2) or the 34-mer (SP34) as in Example 6 except that the peptides were not linked to carrier first. Two rabbits were immunised with each peptide. Periodically, serum was taken from each rabbit and assayed, by ELISA, to determine the production of antipeptide antibodies. The assay plate contained the same peptide as was used as the immunogen, and one microgram of peptide was used per well.

All three peptides were immunogenic, with the stable titre for SP15-2 and SP34 being significantly higher than that for SP21-2. The endpoint dilutions for SP15-2 and SP34 were 1/51,000 and for SP21-2 was 1/6,400. The results are shown graphically in Figure 4, which shows a plot of antiserum titre against homologous synthetic peptide for rabbits immunised with synthetic peptide dimers. Open circles, SP21-2; filled circles, SP15-2; open triangles, SP34. Each point represents the mean from two rabbits. Arrows denote day of injection.

EXAMPLE 14
Synthesis of
    (a) cyclic 17-mer - SP-17 cyclic
C-GA-VDSILGGVATY-GA-C
    (b) linear 35-mer SP35
GA-VDSILGGVATY-GA-GAGAGA-VDSILGGVATY-GAA

SP-11 as described in Example 3 was converted into SP-17 cyclised by adding a CGA residue at each terminus and incubating the resulting 17-mer in ammonium chloride buffer. SP-17 cyclised was purified and characterised as described for SP-21 in Example 1.

SP-11 was also converted to SP-21 (whose preparation is also described in Example 1) by adding three repeat GA units at the amino terminus and a GAAC residue at the carboxy terminus and the resulting SP-21 devoid of its terminal cystein residue was then coupled with the linear version of SP-17 devoid of its terminal cystein residues (SP-15) to give SP-35 that was purified and characterised by the procedure described for SP-21 in Example 1.

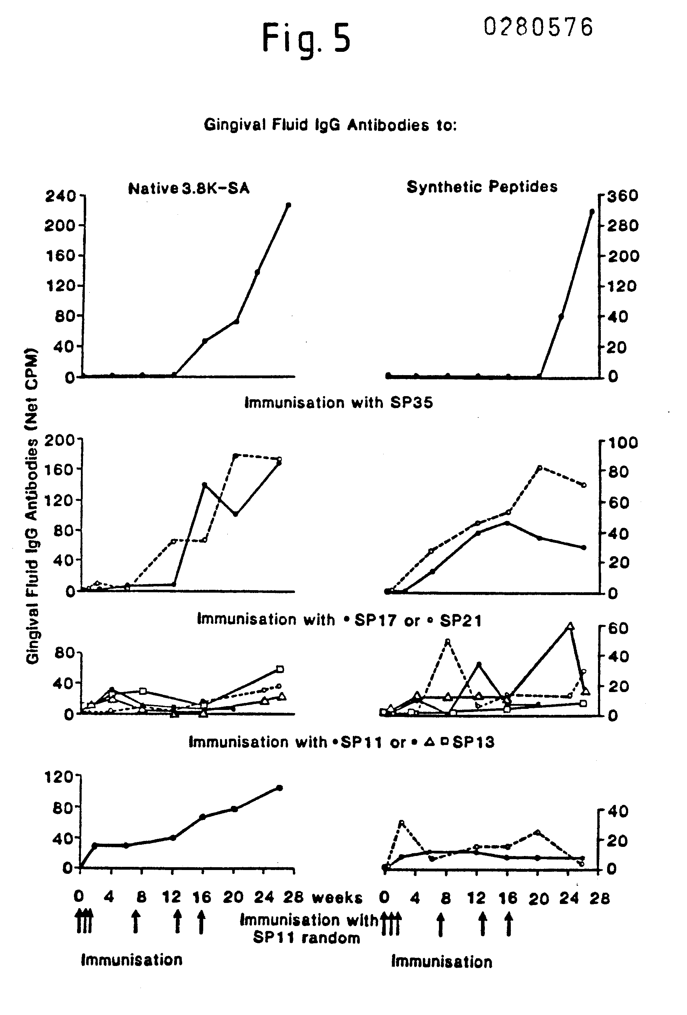

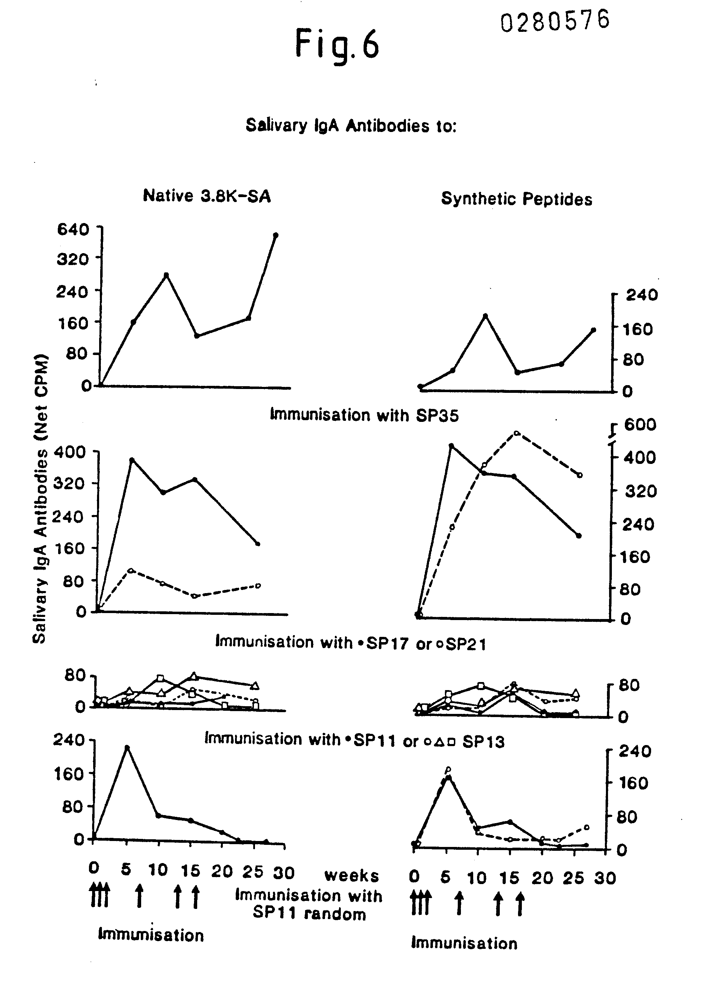

The synthetic peptides SP11, SP11-cyclic (see Example 2), SP17-cyclic and SP35 were applied 5 or 6 times, over a period of 24 weeks to the gingiva of non-human primates. Sequential examination for antibodies over a period of up to 30 weeks by means of a radioimmunoassay revealed that the linear or cyclised SP11 failed to induce a significant increase in gingival fluid IgG or salivary IgA antibodies. In contrast the SP17, SP21 or SP35 induced significant anti-SP as well as anti-native SA antibodies in the gingival fluid and in saliva (Fig. 5 and 6). This dual IgG and secretory IgA immune response was not associated with conventional serum antibody response. Any functional effect of this immune response was tested by offering the monkeys a human type of carbohydrate-enriched diet which leads to colonisation of Streptocossus mutans. Whereas control animals, sham-immunised with a random synthetic peptide CSLFVPSEFS showed increased colonisation of the deciduous teeth by Streptococcus mutans, there was no colonisation with SP17 and a significant reduction in colonisation of animals immunised with the SP21 or SP35. These experiments suggest that local immunisation with synthetic peptide (SP) derived from the amino acid sequences of a streptococcal cell wall antigen (SAI/II) induce a dual local immune response of gingival IgG and salivary IgA antibodies against the synthetic peptide and native streptococcal antigens (SA). These antibodies may be involved in preventing colonisation of Streptococcus mutans which is the principal agent in the development of dental caries.

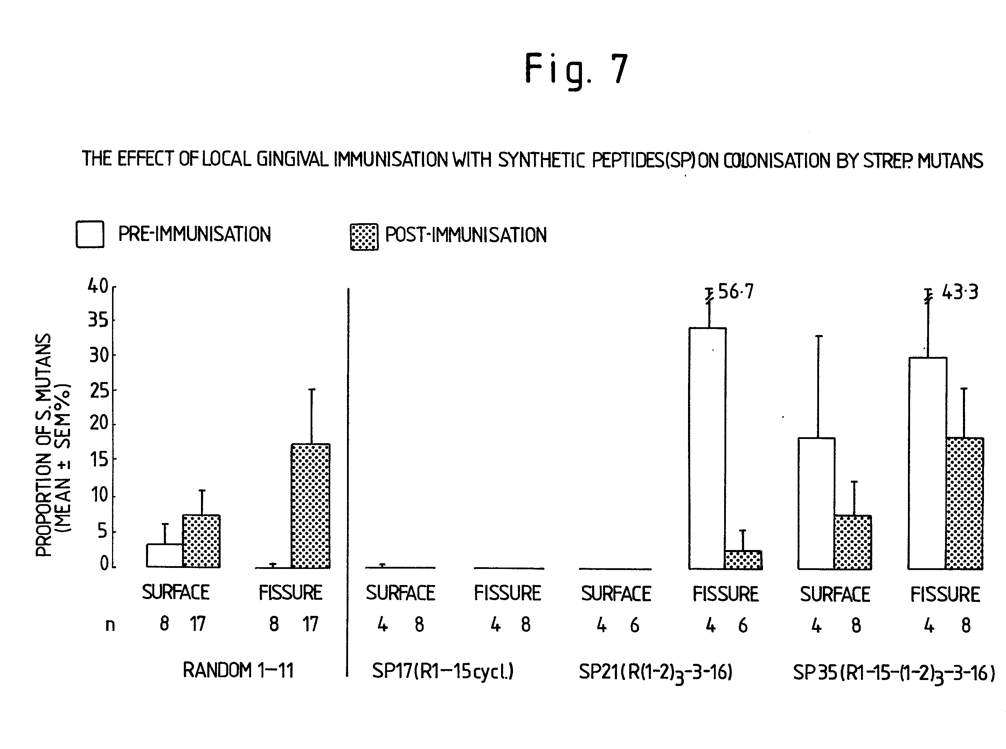

Sequential radioimmunoassay of the gingival fluid IgG and salivary IgA antibodies revealed a significant increase in anti-SP, as well as native anti-SA antibodies, when immunised with SP17, SP21 or SP35 dimer. The development of antibodies differed markedly between salivary IgA which reached optimum levels between 5 and 10 weeks and gingival fluid IgG antibodies which appeared mostly after 10 weeks, with optimum levels being reached by week 26 (Figs. 5 and 6). The higher salivary IgA antibody levels are not comparable with the lower gingival fluid IgG antibodies, because the washing method of collection dilutes the fluid about 100 times. The SP35 consisting of peptide linked linear SP15 and SP20 (without the cysteine residues) was not obviously more immunogenic than any one of the constituent SP17 or SP21. The core SP however, failed to induce a significant increase in gingival fluid IgG or salivary IgA antibodies. This was found with the linear of cyclised SP11, as well as when the latter was covalently linked to PPD or mixed with interferon gamma (Figs. 5 and 6). Sham-immunisation with the random SP11 showed an initial increase in salivary IgA antibodies followed by a decrease, but gingival fluid anti-SA IgG antibodies showed a modest increase which may have reflected a natural immune response due to colonisation by Streptococcus mutans (Fig. 7).

An increase in serum IgG and IgA antibodies was detectable, but the antibody levels were negligible by comparison with systemic immunisation (Table 1).

Experimental Details of Figs. 5 and 6 are as follows:
Figs. 5 and 6 shows results when the control group of 4 sham-immunised macaque monkeys (3 fascicularis and 1 mullata) was immunised by the local oral route, using a random synthetic peptide of 11 amino acid residues which is without any known homology with the synthetic peptide of the invention. The immunised group of 6 Macacca fascicularis and 8 Macacca mulatta were given SP11 or 13 (8 monkeys), SP17 (2 monkeys), SP21 (2 monkeys) and SP35 (2 monkeys). All aminals had the synthetic peptide (SP) applied by means of a Hamilton syringe on 6 separate occasions as indicated by the arrows, except that the SP11 immunised animals were given the SP at week 1, 2, 4, 16 and 24. The concentration of the SP was 10 μg in 150 μl of distilled water and 50% dimethyl sulphoxide (DMSO) and this was applied to the gingival sulci, carefully avoiding any trauma to the gum. To retain the SP in situ silicone rubber trays were prepared and applied to the teeth and gums for 5 minutes after each of the applications of SP. Antibodies to the native 3.8K-SA, SP11, SP17, SP21, SP35 or random 11-mer were tested for IgG antibodies in the gingival fluid (1:5 dilution) and for IgA antibodies in saliva (1:2 dilution) by a solid phase radioassay, using polystyrene wells and $^{125}$I-labelled

affinity purified rabbit anti-monkey IgG (Nordic Pharmaceuticals Ltd.) or rabbit anti-monkey IgA, followed by $^{125}$I-labelled anti-rabbit IgG (Tago Inc. San Francisco), respectively. Gingival fluid washings and pilocarpine (0.5 mg/kg) stimulated saliva were collected at the given intervals, as described previously. The results are expressed as the net mean count per minute, after the counts in the gingival fluid or saliva before immunisation were subtracted.

Fig. 7 show results when the groups of animals described in Figs. 5 and 6 (with the exception of the SP11 linear of cyclised immunised monkeys) were offered a human type of carbohydrate-enriched diet, 2-4 months before the stat of immunisation. Bacterial plaque was collected with sterile probes from the surfaces of the maxillary central incisors and the fissures of the left second deciduous molar before, during and after immunisation. The samples were placed into transport medium and grown on tryptone-yeast extract L-cystine (TYC) medium. The number of colony forming units of Streptococcus mutans were expressed as a percentge of the total colony count on the TYC medium. The data presented are the mean ( ± sem) of 4 to 17 samples, as indicated (n).

EXAMPLE 15

AKKAYEEALAANTAKC     (Peptide 52)
AKADYEAKLAQYEKDLAAC     (Peptide 66)
AYEQELARVQAANAAC     (Peptide 87)
AQYEKDLAAAQAGNAC     (Peptide 94)
CFILSQVPLLGQLSSC     (Peptide 99)
KPRPIYEAKLAQNOK     (Peptide 188)

Using the peptide synthesis techniques including protecting and deprotecting methods, purification and characterisation techniques of Example 1, peptides 52, 56, 87, 94, 99 and 188 were prepared using the relevant amino acids in the relevant order.

Additional protecting groups for the side chains were as follows:

Lys-Clz 2-Chlorobenzyloxycarbonyl (Lysine)
Glu-Bzl Benzyl (Glutamic acid)
Arg-Tos 4-Toluene sulphonyl (Arginine)

These protecting groups also protect the peptide side chains from alkylation or oxidation. They are removed under the same conditions as those described in Example 1.

EXAMPLE 16

Determination of specificity of Example 15 peptides

Peptides from Example 15 were used to immunise Balb/C mice as in Example 6 except that the peptides were not linked to carrier. After five injections over six weeks the animals were bled on week seven and the resulting antibodies were tested for specificity and quantity (titre) by ELISA as in Example 7. Table 1 shows the results as the geometric mean titre for three animals per peptide tested against the peptide they were immunised with.

Confluent cultures of S. mutans 10449 and S. sobrinus 6715 were produced and 0.01 ml aliquots were dried onto nitrocellulose filters. The filters were incubated with a 1:100 dilution of mouse serum, in m-T wash, either before or after immunisation. The filters were washed and incubated with a 1:200 dilution of goat anti-murine antibody conjugated to horseradish peroxidase. Any mouse antibody could then be visualised colorimetrically after the peroxidase was allowed to react with the colorimetric substrate 4-Chloro 1-Naphthol.

Table 1 shows the results. Column 2 lists the geometric mean titre whereas column 3 lists whether Balb/C lymphocytes proliferate when stimulated with the same peptide the animal was immunised with (see Example 8, Figure 3). S.m. denotes whether the bacterium S. mutans is bound by the antipeptide antiserum and S.s. is analogous for S. sobrinus + + is thegreatest colorimetric reaction, + is intermediate, and +/- is slight, when compared to pre-immune values.

## TABLE 1

| Peptide | Titre | T-cell | S.s. | S.m. |
|---|---|---|---|---|
| 52 | 40,000 | + | + | + |
| 66 | 10,000 | + | ++ | ++ |
| 87 | 100,000 | + | + | + |
| 94 | 15,000 | + | +/− | + |
| 99 | 15,000 | + | ++ | ++ |
| 188 | 100,000 | + | ++ | ++ |

**Claims**

1. A synthetic peptide comprising a peptide having amino acid sequences identical or substantially identical to fragments of Antigen I or Antigen I/II or Antigen X, the peptide having amino acid sequences idential or substantially identical to fragments of Antigen I or Antigen I/II or Antigen X being capable of provoking formation in vivo of antibodies that recognise S. mutans antigen (SA).

2. A peptide according to claim 1 containing 6-50 amino acid units.

3. A peptide according to claim 1 or 2 containing the antigenic determinants present in Antigen X.

4. A peptide according to any one of the preceding claims containing 10-34 amino acid units.

5. A peptide according to claim 4 including the sequence VDSILGGVATY or GAGAGAVDSILGGVATYGAAC.

6. A peptide according to any one of claims 1 to 3 including the sequence VDSILGGVATYGAAC and dimers thereof;
dimers of GAGAGAVDSILGGVATYGAAC;
or GAVDSILGGVATYGAAGAVDSILGGVATYGAALC.

7. A peptide according to claim 1 having the sequence:
   AKKAYEEALAANTAKC    (Peptide 52)
   AKADYEAKLAQYEKDLAAC    (Peptide 66)
   AYEQELARVQAANAAC    (Peptide 87)
   AQYEKDLAAAQAGNAC    (Peptide 94)
   CFILSQVPLLGQLSSC    (Peptide 99)
   KPRPIYEAKLAQNQK    (Peptide 188).

8. A peptide according to any one of the preceding claims conjugated to additional protein such that the resulting conjugate is immunogenic.

9. A process for producing a peptide as defined in any one of the preceding claims by building the peptide chain from individual amino acids or peptides thereof by sequentially reacting an N-protected amino acid or peptide thereof with a carboxy protected amino acid or peptide thereof to form amide links and finally removing any remaining protecting groups.

10. A process for producing a peptide as defined in any one of claims 1 to 8, by expressing from a host cell DNA encoding the desired peptide sequence.

11. A vaccine for the control of dental caries comprising a peptide according to any one of claims 1 to 8 in association with a carrier or diluent.

12. A vaccine according to claim 11 in a form suitable for gingival application.

13. An antibody to a peptide according to any one of claims 1 to 8.

14. A monoclonal antibody according to claim 13.

15. A method of producing a vaccine according to claim 11 or 12 by formulating the peptide with a carrier or diluent.

16. A method of producing an antibody according to claim 13 which comprises injecting the peptide into a host animal and recovering antibody from the serum of or other body cells or fluids of the host.

17. A method of producing an antibody according to claim 14 which comprises injecting the peptide into a mouse or other host, fusing spleen cells from the mouse or other host with myeloma cells to produce a hybridoma and recovering antibody from the hybridoma.

18. A passive vaccine for the control of dental caries comprising an antibody according to claim 13 or 14 together with a carrier or diluent.

19. A vaccine according to claim 18 in a form suitable for gingival application.

20. A method of producing a vaccine according to claim 18 or 19 which comprises formulating the antibody with the carrier or diluent.

21. A method for the control of dental caries in a primate which comprises administering to the primate a peptide according to any one of claims 1 to 8 or an antibody according to claim 13 or 14 or a vaccine according to claim 11 or 12 or 18 or 19.

22. A method according to claim 21 wherein the peptide, antibody or vaccine is applied gingivally.

Fig. 1

↓ IMMUNIZATION OF RABBITS
● 11-mer
+ 12-mer (MBS)
○ 13-mer (CYCLIC)
▲ 18-mer
△ 21-mer

0280576

# Fig. 2

A  ANT: 18-mer SERUM

Abs_405

.08

.04

50    200    800    3200

1/DILUTION

△—△ SERUM
●----● SERUM+5µg/ml P3
■—■ SERUM+25µg/ml P3
○—○ SERUM+5µg/ml 18-m

B  ANT: 21-mer SERUM

Abs_405

.08

.04

50    200    800    3200

1/DILUTION

● SERUM
■ SERUM+5µg/ml P3·8
△ SERUM+25µg/ml P3·8
○ SERUM+5µg/ml 21-m

0280576

# Fig. 3

05 04 88

0280576

# Fig. 4

RABBIT RESPONSE TO SYNTHETIC PEPTIDES

Legend:
- 0 SP21-2
- ● SP15-2
- △ SP34
- ↑ INJECTION

Y-axis: Ab TITRE (1E5, 1E4, 1000, 100, 20)

X-axis: WEEKS (0, 5, 10, 15)

## Fig. 5

0280576

Gingival Fluid IgG Antibodies to:

Fig.6

0280576

Salivary IgA Antibodies to:

Native 3.8K-SA

Synthetic Peptides

Immunisation with SP35

Immunisation with •SP17 or ○SP21

Immunisation with •SP11 or ○△□ SP13

Immunisation with
SP11 random

Salivary IgA Antibodies (Net CPM)

weeks

Immunisation

Immunisation

# Fig. 7

THE EFFECT OF LOCAL GINGIVAL IMMUNISATION WITH SYNTHETIC PEPTIDES(SP) ON COLONISATION BY STREP. MUTANS

☐ PRE-IMMUNISATION   ▨ POST-IMMUNISATION

PROPORTION OF S. MUTANS (MEAN ± SEM%)

56·7   43·3

| | SURFACE | FISSURE | SURFACE | FISSURE | SURFACE | FISSURE | SURFACE | FISSURE |
|---|---|---|---|---|---|---|---|---|
| n | 8  17 | 8  17 | 4  8 | 4  8 | 4  6 | 4  6 | 4  8 | 4  8 |

RANDOM 1–11    SP17(R1–15cycl.)    SP21(R(1-2)$_3$-3-16)    SP35(R1-15-(1-2)$_3$-3-16)